# EUROPEAN PATENT SPECIFICATION

(11) **EP 1 264 821 B1**
(45) Date of publication and mention of the grant of the patent: **14.05.2008**
(21) Application number: 01912367.8
(22) Date of filing: 14.03.2001
(51) Int. Cl.: C07C 237/04, C07C 231/24, A61K 31/167, A61P 35/00

(54) **NOVEL CRYSTAL OF STILBENE DERIVATIVE AND PROCESS FOR PRODUCING THE SAME**
NEUES KRISTALLINES STILBEN-DERIVAT UND VERFAHREN ZU DESSEN HERSTELLUNG
NOUVEAU DERIVE DE CRISTAL DE STILBENE ET SON PROCEDE D'OBTENTION

(30) Priority: 17.03.2000 JP 2000076887
(43) Date of publication of application: 11.12.2002
(73) Proprietor: Ajinomoto Co., Inc., Tokyo 104-8315 (JP)
(72) Inventor: TAKAHASHI, Shinichiro Pharmaceu. Research Lab., Kawasaki-shi Kanagawa 210-8681 (JP); SUGAWARA, Yoko, Sagamihara-shi Kanagawa 228-0829 (JP); MATSUEDA, Hiroyuki Pharmaceutical Research Lab., Kawasaki-shi Kanagawa 210-8681 (JP)
(74) Representative: Nicholls, Kathryn Margaret
(86) International application number: PCT/JP2001/002028
(87) International publication number: WO 2001/068587

(56) References cited:
- EP-A- 0 731 085
- OHSUMI K. ET AL.: 'Synthesis and antitumor activities of amino acid prodrugs of amino-combretastatins' ANTI-CANCER DRUG DESIGN vol. 14, no. 6, 1999, pages 539 - 548, XP002938076

## Description

The present invention relates to a novel stilbene derivative crystal. More specifically, the invention relates to a Type IV crystal of (Z)-N-[2-methoxy-5-[2-(3,4,5-trimethoxyphenyl)vinyl]phenyl]-L-serinamide hydrochloride, which is useful as an effective ingredient of a carcinostatic agent (anticancer agent), and a method for producing the same.

Stilbene derivatives of a specific structure have an anticancer activity (action), and are useful compounds as effective ingredients of carcinostatic agents (see Japanese Patent Kokai Publication JP-A-8-301831, International Publication WO 99/51246, and the like).

Taking in account that (Z)-N-[2-methoxy-5-[2-(3,4,5-trimethoxyphenyl)vinyl]phenyl]-L-serinamide hydrochloride represented by the following structure (structural) formula (1) : 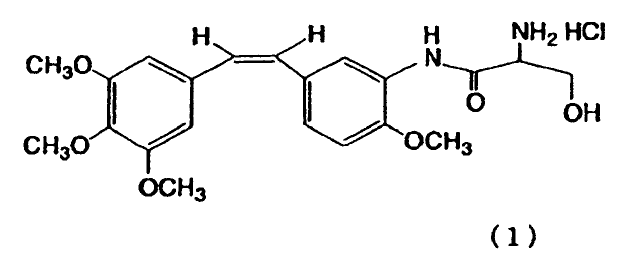 is particularly excellent as an effective component (ingredient) for the development of a pharmaceutical agent among a great number of stilbene derivatives with a carcinostatic action(s), the present inventors have made investigations about the crystallization thereof. The inventors have found that the crystal of the compound as recovered by a general method (Hereinafter, the crystal type recovered by the general method is referred to as "Type I", and the crystal of this type is referred to as "Type I crystal".) adsorbs water (water vapor) in roughly (approximately) proportional to water vapor pressure (relative humidity) to adsorb water depending on the surrounding humidity. Additionally, the inventors have found that the quantity of the adsorbed water is fairly large. Such crystal unstable in the presence of water is not preferable for drug preparation. For the production of drugs and dosage forms (pharmaceutical preparations) thereof, a stable crystal with less (very little) water adsorption and less (very little) water (water vapor) adsorption up to around a relative humidity of 80 % is preferable.

The crystallisation of the compound of formula (1), using a mixed solvent of ethanol and ethyl acetate, is described in Ohsumi, K. et al., Anti-Cancer Drug Design, 1999, vol 14, No. 6, pp. 539-548.

It is a purpose of the present invention to provide a crystal of the stilbene derivative represented by the structural formula (1), which is at such an extremely low water (water vapor) adsorption level as described above, namely a crystal stable to water (moisture), a method for producing the crystal and further a use thereof.

In order to overcome the problems as described above and attain the purpose of the present invention, the present inventors have made investigations about crystals of (Z)-N-[2-methoxy-5-[2-(3,4,5-trimethoxyphenyl)vinyl]phenyl]-L-serinamide hydrochloride as represented by the structural formula (1) described above, to find a novel stable crystal thereof to water (moisture) (Hereinafter, the crystal is referred to as "Type IV crystal".), which differs from the crystal type (Type I) obtained by the general method. As specifically shown in the X ray powder diffraction pattern, the Type IV crystal obtained in accordance with the present invention is a novel crystal apparently different from the crystal type obtained by the general method. The present inventors have found that the novel crystal is so stable to water (moisture) that the crystal adsorbs extremely less water (water vapor) up to around a relative humidity of 80 % and that a trace content of water in a solvent used for the crystal formation (crystallization) thereof is a factor determining such crystal type. Based on these various findings, the present invention has been achieved.

That is, the present invention relates to the novel crystal, more specifically, the Type IV crystal of (Z)-N-[2-methoxy-5-[2-(3,4,5-trimethoxyphenyl)vinyl]phenyl]-L-serinamide hydrochloride represented by the structural formula (1), which is a novel crystal (Type IV crystal) differing from that of the crystal recovered (obtained) by the general method, as shown in the X ray powder diffraction pattern and which has peaks at least at 11.1 ° 13.4 °, 14.2 °, 18.7 °, 19.4 °, 22.5 ° and 23.4 ° (2θ) in the X ray powder diffraction pattern. In other aspects, furthermore, the present invention relates to a carcinostatic agent comprising the Type IV crystal, and a method for producing the crystal.

More specifically, the present invention encompasses the following contents.
(1) The crystal of the present invention described above, having peaks in the X ray powder diffraction pattern which contain those at 11.1 °, 13.4 °, 14.2 °, 18.7 °, 19.4 °, 22.5 ° and 23.4 ° (2θ).

The crystal of the present invention may adsorb water (water vapor) at the level of less than 20 ml/g (preferably, less than 15 ml/g or so) at a relative humidity of 80 %, on a prepared water (water vapor) adsorption isotherm (isothermal line) (see Mitsuiki et al., J. Agric. Food Chem., vol. 46, No. 9, pages 3528-3534, 1998).

Here, the water (water vapor) adsorption level represents the water vapor volume occupying one gram crystal [ml/g] at 0 °C and one atmospheric pressure.

Further, preferably, the water (water vapor) adsorption levels at relative humidity of 40, 60 and 80 % are less than about 8 ml/g (more preferably, less than about 6 ml/g), less than about 16 ml/g (more preferably, less than about 9 ml/g) and less than about 20 ml/g (more preferably, less than about 15 ml/g), respectively.

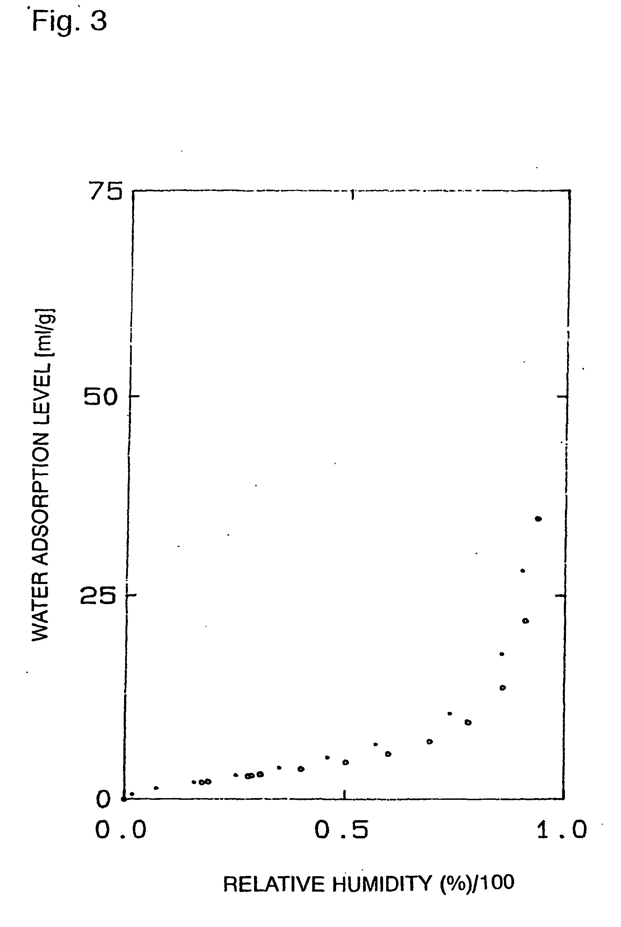

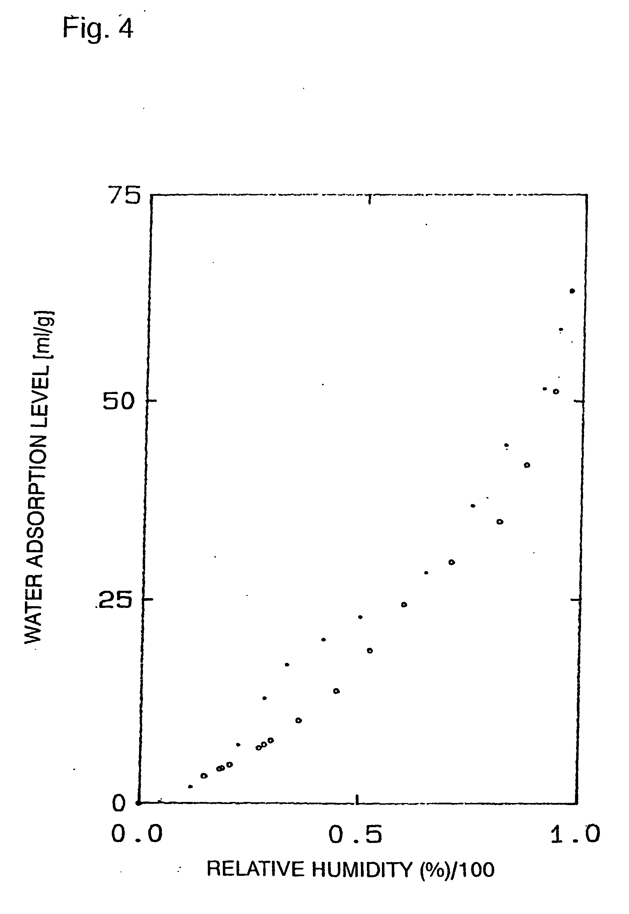

Water (water vapor) adsorption isotherms both in adsorption and desorption processes (Water vapor adsorption levels measured by the method for measuring water (moisture content) when humidity increases and decreases to dryness) are shown in Figs. 3 and 4. Preferably, a smaller level of the resulting two levels satisfies the above numerical range.

The crystal of the present invention can be crystallized using a solvent, which may be a mixed solvent, containing water at 2 % by weight at the maximum, preferably a mixed solvent of methanol-isopropyl acetate with no water contained substantially.
[2]

A carcinostatic agent (anticancer agent) such as oral dosage form or parenteral dosage form (injections, etc.) containing the Type IV crystal described above or being produced by using the Type IV crystal described above (which may contain carrier(s) used in the method for producing a dosage form (a pharmaceutical preparation).

Thus the Type IV crystals can be used as a carcinostatic agent, for instance in the therapeutic treatment, amelioration (improvement), progress prevention, prophylaxis (prevention), etc. of cancer, comprising administration of the crystal into an animal such as a human in need of the carcinostatic effect.

Incidentally, depending on the symptom and the like of a patient or the like, the dose of the crystal in this case can appropriately be determined, based on the method for use of routine carcinostatic agents and known methods for use of the carcinostatic agents. The carrier for use in the production of dosage forms thereof can be determined appropriately on the basis of routine pharmacological methods.
[3]

A method for producing a crystal of (Z)-N-[2-methoxy-5-[2-(3,4,5-trimethoxyphenyl)vinyl]phenyl]-L-serinamide hydrochloride represented by the structural formula (1) described above containing a crystallization step therefor, comprising the crystallization step with a crystallization solvent containing water at 2 % by weight at the maximum (at 2 % by weight or less) for the crystallization of the Type IV crystal thereof.

Concerning the water content of the crystallization solvent, a solvent containing water at more preferably approximately 1 % by weight at the maximum (approximately 1 % by weight or less), and still more preferably approximately 0.5% by weight (approximately 0.5 % by weight or less), and most preferably a solvent with substantially no water can be used. In case that a mixture solvent (a mixed solvent) of plural solvents is used as the crystallization solvent, the crystallization solvent is preferably at such a small amount of water content in total as described above. Accordingly, in case that a combination of good solvent(s) and poor solvent(s) is used for re-precipitation, for example, the whole mixture system of the good solvent (s) and the poor solvent (s) to be used may be adjusted to the small amount of water content described above. Here, a combination of the good solvent (s) and the poor solvent(s) to be used for re-precipitation is also encompassed within the range of the term "mixture solvent" described above, according to the method of the present invention described above.

The method for producing the crystal (Type IV crystal) described above, where said solvent to be used at the crystallization step is a combination (mixture solvent; mixed solvent) of good solvent (s) and poor solvent (s) for the compound (1).

The method for producing the crystal (Type IV crystal) described above, where said good solvent(s) include at least one compound selected from alcohols, chloroform, acetone and acetonitrile and said poor solvent(s) include at least one compound selected from acetate ester (acetic acid ester), ethers, saturated hydrocarbons and cyclic saturated hydrocarbons.

In the method for producing the crystal (Type IV crystal) described above, the crystallization step is preferably via (through) re-precipitation method.

### Brief Description of the Drawings

[Fig. 1]

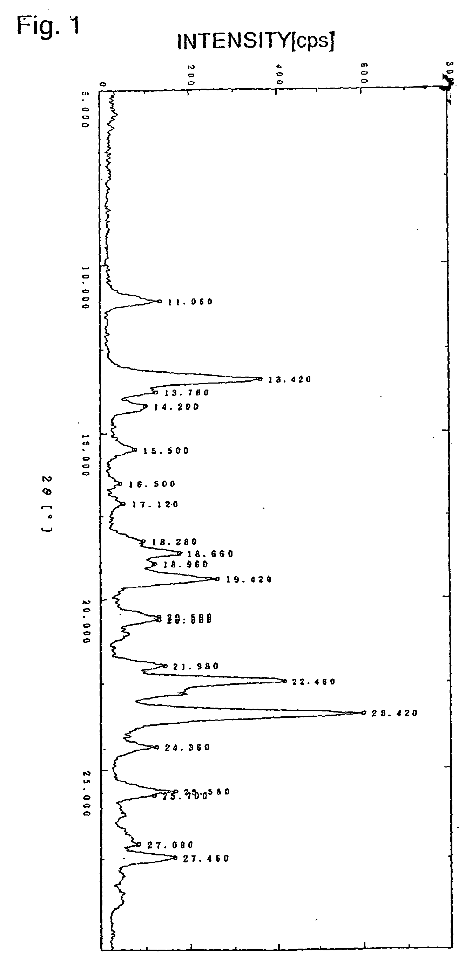

   Fig. 1 shows a X ray powder diffraction pattern of the novel Type IV crystal of the present invention, obtained (recovered) in Example 1.
[Fig. 2]

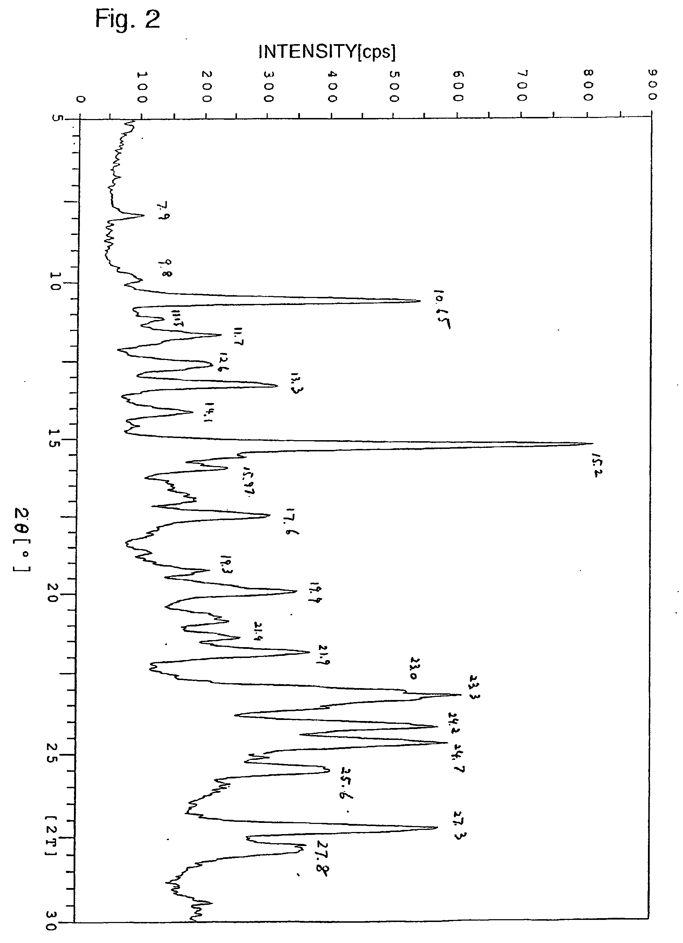

   Fig. 2 shows a X ray powder diffraction pattern of the crystal (Type I crystal) obtained by the general method, which is obtained in Comparative Example 1.
   In Figs. 1 and 2 described above, a horizontal axis represents 2θ (degrees) and a vertical axis represents intensity (CPS).
[Fig. 3]
   Fig. 3 shows a water (water vapor) adsorption isotherm (isothermal line) of the Type IV crystal of the present invention, obtained in Example 1.
[Fig. 4]
   Fig. 4 shows a water (water vapor) adsorption isotherm (isothermal line) of the crystal (Type I crystal) obtained by the general method, which is obtained in Comparative Example 1.
   In Figs. 3 and 4, the vertical axis represents water (water vapor) adsorption level [ml/g] (water vapor volume occupying one gram of crystal at 0 °C and one atmospheric pressure) and the horizontal axis represents relative humidity (%) /100. Furthermore, open circle(○) represents a value when humidity is increased from dryness and solid circle(●) represents a value when humidity is decreased to dryness.

### Mode for Carrying out the Invention

The mode of carrying out the present invention is now described below.

The Type IV crystal of (Z)-N-[2-methoxy-5-[2-(3,4,5-trimethoxyphenyl)vinyl]phenyl]-L-serinamide hydrochloride represented by the structural formula (1) described above in accordance with the present invention can easily be produced at a crystallization step (for example, crystallization, re-precipitation, etc.) using a solvent containing water at about (approximately) 2 % by weight at the maximum, most preferably a solvent with no water contained substantially as the crystallization solvent.

The water content is preferably about (approximately) 2 % by weight at the maximum, more preferably about 1 % by weight at the maximum, and further more preferably about 0.5 % by weight at the maximum, to the total crystallization solvent to be used (for example, a total sum of good solvent(s) and poor solvent (s) in case of re-precipitation using a combination of the good solvent(s) first dissolving the intended compound and the poor solvent(s) subsequently crystallizing the intended compound therefrom, respectively). Most preferably, the crystallization step is carried out, using a solvent with no water contained substantially.

Any crystallization step can be used with no specific limitation. The crystallization step includes any crystallization means capable of dissolving the intended compound in a solvent to crystallize the compound, such as concentration crystallization (crystallization under concentration), cooling crystallization (crystallization under cooling), neutralization crystallization (crystallization under neutralization), and crystallization via (through) re-precipitation.

Here, the method for producing the crystal in accordance with the present invention includes a step of crystallizing the Type IV crystal in the hydrochloride form. The Type I crystal obtained (recovered) by the general method can be used simply as a starting material for the crystallization step. However, the Type I crystal is not necessarily used as the starting material therefor, provided that crystallization is performed in the form of the Type IV crystal at the crystallization step. Therefore, the compound in the form containing the free form or the salt form, which may include the hydrochloride salt form, such as the free form, the salt forms other than hydrochloride salt form, reaction solutions for the synthesis of the intended compound, etc. can be used for a starting material in the crystallization step. It is needless to say to require a salt formation step for converting the free form into the hydrochloride salt form in case of the free form, or a step for converting other salt form(s) into the hydrochloride salt form in case of such other salt form(s).

Solvents known for use or usable for such organic compound salts can be used as the crystallization solvent. An appropriate crystallization solvent can be selected at a preliminary simple test of solubility for the intended compound. The crystallization solvent for use in this case may be a single solvent or a mixture of plural solvents (mixture solvent; mixed solvent). Preferably, a mixture solvent can be used, which is prepared by mixing together a solvent sufficiently dissolving the intended compound (good solvent) and a solvent hardly dissolving the intended compound, which however can be dissolved in the good solvent (poor solvent) at appropriate volumes. Additionally, plural solvents can be used as good solvents, while plural solvents can be used as poor solvents. In this case, preferably, these plural solvents are miscible together.

For the crystallization via re-precipitation method, the selection of the good solvent(s) and the poor solvent(s) and their volumes thereof are particularly important. Based on preliminary experiments of solubility therefor and the like, appropriately, the optimal conditions can easily be selected. For example, a combination of methanol and isopropyl acetate is particularly preferable.

The good solvent preferably includes alcohols such as methanol, ethanol, n-propyl alcohol and isopropyl alcohol, chloroform, acetone, acetonitrile and the like. It is needless to say that one or more thereof can be used.

The poor solvent preferably includes acetate ester such as methyl acetate, ethyl acetate, isopropyl acetate, n-propyl acetate, n-butyl acetate, and isobutyl acetate; ethers such as diethyl ether; hydrocarbons such as hexane and cyclohexane. It is needless to say that one or more thereof can be used.

The crystal produced in accordance with the present invention (Type IV crystal) distinctly differs from the crystal (Type I crystal) obtained by the general method, in view of the X ray powder diffraction pattern (see Figs. 1 and 2). In other words, the inventive crystal characteristically has peaks containing at least those at 13.4 °, 18.7°, 19.4 °, and 22.5 ° (2θ), and preferably those at 11.1 °, 13.4 °, 14.2 °, 18.7 °, 19.4 °, 22.5 ° and 23.4 ° (2θ).

Furthermore, the crystal produced in accordance with the present invention (the inventive product) is compared with the crystal produced by the general method, based on water (water vapor) adsorption isotherm (water adsorption constant temperature line) (see Figs. 3 and 4). While the Type I crystal of the related art (prior art) adsorbs much water (moisture) in roughly (approximately) proportional to water vapor pressure, the inventive product (Type IV crystal) adsorbs so extremely less water (water vapor) up to around a relative humidity of 80 % that the inventive product is extremely stable to (in the presence of) the water (moisture). Consequently, the inventive product is very useful for the production of drugs and dosage forms (pharmaceutical preparations), compared with the crystal produced by the general method.

### Preferred Mode for Carrying out the Invention

The present invention will now be described in Examples and Comparative Example in more detail below. However, these are only typical examples. Thus, the present invention encompasses these Examples but is not limited to these Examples.

### (Examples and Comparative Example)

To an acetonitrile solution of the free-form compound prepared according to the description of the specification in the Japanese Patent Kokai Publication JP-A-8-301831, Example 4, was added a 10 % solution of hydrogen chloride in methanol. The deposited crystals were filtered, recovered, and dried to obtain the crystals (1.2 g) of the stilbene derivative ((Z)-N-[2-methoxy-5-[2-(3,4,5-trimethoxyphenyl)vinyl]phenyl]-L-serinamide 1-hydrochloride salt) as produced by conversion into the hydrochloride salt form from the free form thereof. The crystals were dissolved in solvents prepared by preliminarily adding water at volumes shown in Table 1 to methanol (anhydrous methanol) (2 ml), followed by addition of isopropyl acetate (anhydrous isopropyl acetate) (6 ml) at 5 °C to crystallize (re-precipitate) the stilbene derivative, which was then left to stand at 5 °C. In 5 minutes and one day after the addition of the isopropyl acetate, the individually separated stilbene derivative crystals were subjected to a powder X-ray diffractometry for recovery of their patterns. The crystals of Examples 1 and 2 showed X ray powder diffraction patterns differing from the pattern of the stilbene derivative crystal (crystal recovered by the general method) used as the starting material in the Examples. The crystal with the X ray powder diffraction pattern of the general crystal recovered by the general method is referred to as "Type I crystal" (see Fig. 2). The crystal with a novel X ray powder diffraction pattern differing from that of the crystal recovered by the general method is referred to as "Type IV crystal" (see Fig. 1). These results are shown in Table 1.

**[Table 1]**

| Relation between water content in crystallization solvent and X ray powder diffraction pattern (crystal type) | | | |
|---|---|---|---|
| Crystallization example | Water(ml) added to methanol (2ml) | Crystal type | |
| | | 5 minutes later | 1 day later |
| Example 1 | 0 | IV | IV |
| Example 2 | 0.025 | IV | IV |
| Comparative Example 1 | 0.2 | I | I |

As apparently seen from the results, the crystal recovered by reprecipitation after dissolution in 2 ml of methanol with addition of 0.2-ml water was Type I crystal with the same X ray powder diffraction pattern as that of the starting material crystal used in the Examples, whereas the crystals recovered by reprecipitation after dissolution in methanol with no water content or with addition of 0.025-ml water were novel crystals with a X ray powder diffraction pattern never observed for the crystal recovered by the general method (see Fig.1).

Further, both Types I and IV had an identical melting point by melting point measurement (183 to 186 °C).

As to the water content in all the crystallization solvent (methanol and isopropyl acetate), still further, it was confirmed that Type IV crystal could be recovered with a solvent at a water content less than 2 % by weight.

### (Preparation of water adsorption isotherm (water adsorption constant temperature line))

After the Type IV crystal recovered above was dried overnight in vacuum at 60 °C, the water adsorption level of the crystal was measured at a constant temperature of 25 °C by a fully automatic water adsorption analyzer (BELSORP-18: Nippon Bell K.K.) (see Mitsuiki et al., J. Agric. Food Chem., vol.46, No. 9, pages 3528-3534, 1998). The results are shown in Fig. 3.

The water adsorption level of the Type I crystal was also measured in the same manner as above. The results are shown in Fig. 4.

These results indicate that the Type IV crystal adsorbed extremely less water up to around a relative humidity of 80 %, while the Type I crystal adsorbed water in roughly proportional to relative humidity (water vapor pressure).

Additionally, with respect to the Type IV crystal, the X ray powder diffraction pattern of the Type IV crystal did not change at a relative humidity of 80 % or less, involving no crystal structure change, which complies with the water (water vapor) adsorption property of the Type IV crystal.

As apparently seen from the aforementioned results, the novel Type IV crystal recovered in accordance with the present invention has so excellent water adsorption property that the crystal is highly stable to water and is very useful for the production of drugs and dosage forms (pharmaceutical preparations).

### Effect of the Invention

The Type IV crystal of the stilbene derivative as represented by the structural formula (1) described above in accordance with the present invention is more stable to (in the presence of) water than the crystal (Type I crystal) recovered by the general method, such that the Type IV crystal adsorbs extremely less water (water vapor) up to around a relative humidity of 80 % and has excellently great properties for the production of drugs and dosage forms (pharmaceutical preparations).

At a crystallization step in a solvent with no water contained substantially, the Type IV crystal with such great properties can be produced easily. Thus, the present invention is highly useful industrially.

## Claims

1. A Type IV crystal of (Z)-N-methoxy-5-[2-(3,4,5-trimethoxyphenyl)vinyl[phenyl]-L-serinamide hydrochloride, which has an X ray powder diffraction pattern with peaks at 11.1°, 13.4°, 14.2°, 18.7°, 19.4°, 22.5°, and 23.4° (2θ).

2. A carcinostatic agent comprising the crystal according to claim 1 and, optionally, a pharmaceutically acceptable carrier.

3. A method for producing a stilbene derivative (Z)-N-[2-methoxy-5-[2-(3,4,5-trimethoxyphenyl)vinyl]phenyl]-L-serinamide hydrochloride in crystalline form, comprising a crystallization step using a crystallization solvent containing water at 2% by weight at the maximum to crystallize the Type IV crystal thereof.

4. The method according to claim 3, where said solvent used at the crystallization step is a combination solvent of good solvent(s) and poor solvent(s) for said compound.

5. The method according to claim 4, where said good solvent(s) is at least one compound selected from alcohols, chloroform, acetone and acetonitrile and said poor solvent(s) is at least one compound selected from acetate ester, ethers, saturated hydrocarbons, and cyclic saturated hydrocarbons.

6. The method according to claim 3, where said solvent is methanol-isopropyl acetate with no water contained substantially.

7. The method according to claim 3, where said crystallization step is via re-precipitation.

## Patentansprüche

1. Typ IV-Kristall von (Z)-N-[2-Methoxy-5-[2-(3,4,5-trimethoxyphenyl)vinyl[phenyl]-L-serinamidhydrochlorid, welcher ein Röntgenstrahlbeugungsmuster mit Peaks bei 11,1°, 13,4°, 14,2°, 18,7°, 19,4°, 22,5° und 23,4° (2θ) aufweist.

2. Carcinostatisches Mittel, welches den Kristall nach Anspruch 1 und gegebenenfalls einen pharmazeutisch geeigneten Träger enthält.

3. Verfahren zum Herstellen des Stilbenderivats (Z)-N-[2-Methoxy-5-[2-(3,4,5-trimethoxyphenyl)vinyl[phenyl]-L-serinamid-hydrochlorid in kristalliner Form, welches eine Kristallisationsstufe unter Einsatz eines höchstens 2 Gew.-% Wasser enthaltenden Kristallisationslösungsmittels umfasst, um den Typ IV-Kristall davon zu kristallisieren.

4. Verfahren nach Anspruch 3, wobei das in der Kristallisationsstufe eingesetzte Lösungsmittel ein Kombinationslösungsmittel eines guten Lösungsmittels oder mehrerer guter Lösungsmittel und eines schwachen Lösungsmittel oder mehrerer schwacher Lösungsmittel für die Verbindung ist.

5. Verfahren nach Anspruch 4, wobei das eine oder die mehreren guten Lösungsmittel mindestens eine Verbindung ist/sind, die unter Alkoholen, Chloroform, Aceton und Acetonitril ausgewählt ist, und das eine oder die mehreren schwachen Lösungsmittel mindestens eine Verbindung ist/sind, die unter Acetatester, Ethern, gesättigten Kohlenwasserstoffen und cyclischen gesättigten Kohlenwasserstoffen ausgewählt ist.

6. Verfahren nach Anspruch 3, wobei das Lösungsmittel Methanolisopropylacetat ist, das im wesentlichen kein Wasser enthält.

7. Verfahren nach Anspruch 3, wobei die Kristallisationsstufe über Umfällung abläuft.

## Revendications

1. Cristal de type IV d'hydrochlorure de (Z)-N-[2-méthoxy-5-[2-(3,4,5-triméthoxyphényl)vinyl][phényl]-L-sérinamide, lequel présente un motif de diffraction de poudre aux rayons X avec des pics à 11,1°, 13,4°, 14,2°, 18,7°, 19,4°,22,5° et 23,4° (2θ).

2. Agent cancérostatique comprenant le cristal selon la revendication 1 et, facultativement, un support pharmaceutiquement acceptable.

3. Procédé pour la production d'un dérivé stilbène d'hydrochlorure de (Z)-N-[2-méthoxy-5-[2-(3,4,5.triméthoxyphényl)vinyl]phényl]-L-sérlnamide dans une forme cristalline comprenant une étape de cristallisation utilisant un solvant de cristallisation contenant de l'eau à 2 % en poids au maximum pour cristalliser le cristal de type IV de celui-ci.

4. Procédé selon la revendication 3, dans lequel ledit solvant utilisé dans l'étape de cristallisation est un solvant de combinaison d'un(de) bon(s) solvant(s) et d'un(de) solvant(s) médiocre(s) pour ledit composé.

5. Procédé selon la revendication 4, dans lequel ledit(lesdits) bon(s) solvant(s) est(sont) au moins un composé choisi parmi des alcools, le chloroforme, l'acétone et l'acétonitrile et ledit(lesdits) solvant(s) médiocre(s) est(sont) au moins un composé choisi parmi un ester d'acétate, des éthers, des hydrocarbures saturés et des hydrocarbures saturés cycliques.

6. Procédé selon la revendication 3, dans lequel ledit solvant est l'acétate de méthanoi-isopropyle sans pratiquement d'eau contenue.

7. Procédé selon la revendication 3, dans lequel ladite étape de cristallisation se fait via une re-précipitation.
